# FASCICULE DE BREVET EUROPEEN

(11) **EP 3 716 097 B1**
(45) Date de publication et mention de la délivrance du brevet: **14.08.2024**
(21) Numéro de dépôt: 20165099.1
(22) Date de dépôt: 24.03.2020
(51) Int. Cl.: G06F 16/9035

(54) **PROCÉDÉ D'ACQUISITION DE DONNÉES CAPTURÉES PAR UN MODULE DE CAPTURE EMBARQUÉ DANS UN ENGIN MOBILE SUIVANT UNE TRAJECTOIRE PRÉDÉTERMINÉE, PROGRAMME D'ORDINATEUR ET DISPOSITIF CORRESPONDANTS**
VERFAHREN ZUR ERFASSUNG VON DATEN, DIE VON EINEM ERFASSUNGSMODUL ERFASST WERDEN, DAS IN EINEM MOBILEN GERÄT NACH EINEM VORGESTELLTEN TRAJEKTOR, EINEM ENTSPRECHENDEN COMPUTERPROGRAMM UND EINEM GERÄT EINGEFASST IST
METHOD FOR ACQUIRING DATA CAPTURED BY A CAPTURE MODULE EMBEDDED IN A MOBILE DEVICE FOLLOWING A PREDETERMINED TRAJECTORY, CORRESPONDING COMPUTER PROGRAM AND DEVICE

(30) Priorité: 25.03.2019 FR 1903097
(43) Date de publication de la demande: 30.09.2020
(73) Titulaire: ALSTOM Holdings, 93400 Saint-Ouen-sur-Seine (FR)
(72) Inventeur: BONNEVAY, Vincent, 69008 LYON (FR); ANDRE, Hubert, 69100 VILLEURBANNE (FR)
(74) Mandataire: Lavoix

(56) Documents cités:
- EP-A1- 2 881 710
- US-A1- 2007 058 048
- US-A1- 2018 054 559

## Description

La présente invention concerne le domaine des engins mobiles, par exemple un train, embarquant un capteur d'images (caméra), un capteur radar et un capteur lidar, propre à capturer des données relatives à l'environnement du train. De tels trains comprennent généralement un organe de traitement des données capturées configurés pour effectuer des traitements sur les données capturées, de type détection d'obstacles etc., en vue de déclencher, par exemple de manière automatique, un ralentissement ou un arrêt d'urgence, ou toute autre action.

La présente invention concerne plus particulièrement un procédé d'acquisition de données, via un module électronique d'acquisition, lesdites données étant capturées par trois capteurs de données d'images choisis parmi : une caméra, un LIDAR et un RADAR qui sont embarqués sur un engin mobile et qui délivrant à chaque instant d'acquisition de chaque capteur un ensemble de données correspondant à la zone d'acquisition de chaque capteur, selon lequel l'engin mobile suit une trajectoire prédéfinie.

Nombre de données ainsi capturées se révèlent difficilement exploitables du fait de données contradictoires, des problèmes d'exposition ou d'exploitation des capteurs notamment en ce qui concerne les images prises juste avant l'entrée d'un tunnel qui généralement ne montrent rien de la trajectoire qui se trouvent dans un halo noir correspondant au tunnel ; ou des difficultés d'exploitation des données capturées par un capteur lidar dans un environnement brumeux, poussiéreux ou lors de précipitations, etc.

Par ailleurs, il existe un besoin de diminuer encore le temps et la ressource de calcul nécessaires au traitement de telles images.

US 2007/058048 A1 décrit un système de photographie comportant plusieurs unités de prise de vue pour un engin mobile. Une unité de commande prédit l'état environnant l'engin sur la base d'informations de carte ou de position et, lorsque l'engin pénètre dans un tunnel, émet une instruction de commande à l'unité de prise de vue afin d'augmenter la quantité d'exposition du dispositif de prise de vue.

Le but de l'invention est alors de proposer un procédé qui permette d'améliorer la situation. A cet effet, suivant un premier aspect, l'invention propose un procédé conforme à la revendication 1 du jeu de revendications annexé.

L'invention permet ainsi de limiter l'analyse/l'acquisition des données capturées (images), à une portion réduite (celle d'intérêt) afin de diminuer le temps et/ou les ressources de traitement et d'optimiser les valeurs des paramètres de capture du ou des modules d'acquisition au vu de ce qu'il sera utile d'exploiter.

Dans des modes de réalisation, le procédé suivant l'invention est conforme à l'une des revendications 2 à 6 du jeu de revendications annexé.

Suivant un deuxième aspect, la présente invention propose un programme d'ordinateur comportant des instructions logicielles qui, lorsqu'elles sont exécutées par un ordinateur, mettent en oeuvre un procédé tel que défini ci-dessus.

Suivant un troisième aspect, la présente invention propose un module électronique d'acquisition de données conforme à la revendication 8 du jeu de revendications annexé.

Suivant un quatrième aspect, la présente invention propose un véhicule adapté pour suivre une trajectoire prédéfinie, comprenant le module électronique d'acquisition de données selon l'invention.

Ces caractéristiques et avantages de l'invention apparaîtront à la lecture de la description qui va suivre, donnée uniquement à titre d'exemple, et faite en référence aux dessins annexés, sur lesquels :
[Fig 1] la figure 1 représente une vue d'un système d'acquisition d'images dans un mode de réalisation de l'invention ;
[Fig 2] la figure 2 est un organigramme d'étapes mises en oeuvre dans un mode de réalisation de l'invention ;
[Fig 3] la figure 3 est une vue illustrant un découpage en sous-zones dans un mode de réalisation de l'invention.

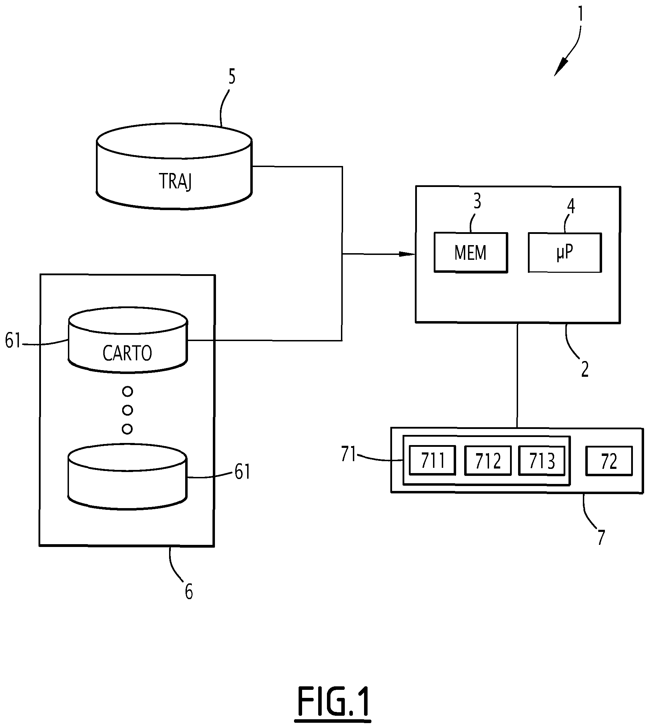

La figure 1 représente un système 1 d'acquisition de données mesurées/capturées par des capteurs d'observation de l'environnement d'un véhicule.

Ce système 1 d'acquisition est adapté pour optimiser l'obtention et l'exploitation de données capturées depuis des capteurs de données choisis parmi un(e) ou des caméra(s), un ou des Lidar(s) (de l'anglais Laser Détection And Ranging), et un ou des radar(s) embarqué(s) dans un train (non représenté). Ce système d'acquisition permet de rendre les données capturées, notamment les images capturées, plus exploitables et/ou de diminuer le temps ou les ressources de calcul nécessaires à l'exploitation de ces données capturées notamment de ces images.

Le train est par exemple piloté par un dispositif de conduite automatique autonome, ou par un conducteur éventuellement assisté par un tel dispositif de conduite automatique.

Le train suit une trajectoire et vitesse prédéterminées entre un point de départ et un point d'arrivée, comportant éventuellement des points d'arrêt intermédiaires, sur des rails.

Dans la description le véhicule présenté est un train, mais ce véhicule est plus généralement propre à être tout type de véhicule, notamment un véhicule guidé, notamment un véhicule ferroviaire.

Le système 1 d'acquisition comprend un bloc de contrôle 2, une mémoire 5 de trajectoire, nommée ci-après mémoire TRAJ 5, un ensemble 6 de sources d'informations et un module de délivrance de données 7.

La mémoire TRAJ 5 comporte les données de définition de la trajectoire du train entre son point de départ et son point d'arrivée, correspondant donc au tracé des rails, sous la forme par exemple des coordonnées géographiques (par exemple sous la forme longitude, latitude, altitude) de points réalisant un échantillonnage de la trajectoire.

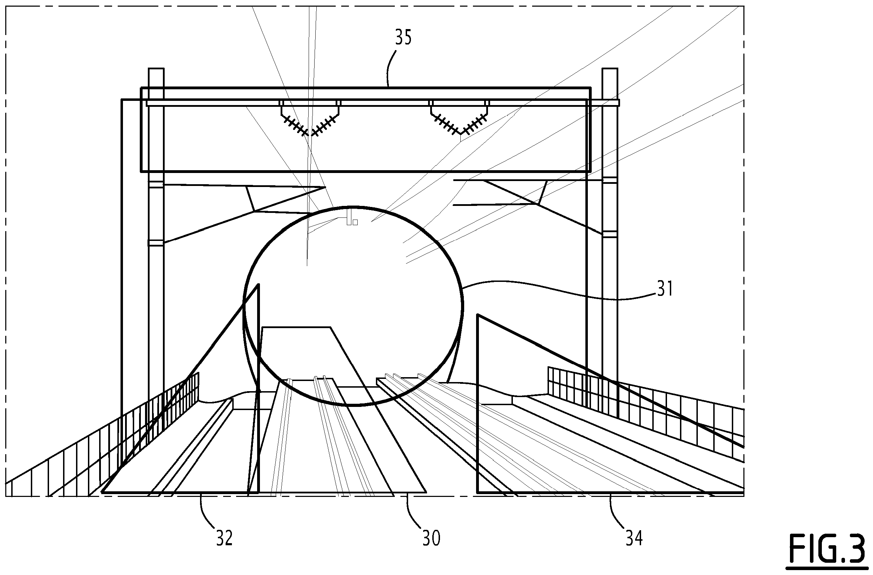

La figure 3 illustre une image prise depuis un capteur de données, notamment une caméra 711, c'est-à-dire depuis un point de la trajectoire du train ; elle montre une voie de rails à gauche correspondant à la trajectoire suivie depuis le point de capture de l'image, par le train, et l'entrée d'un tunnel, au sein duquel la voie ferrée passe.

L'ensemble 6 de sources d'informations comprend une ou plusieurs bases de données 61, dont une base de données nommée ci-après CARTO 61.

La base de données CARTO 61 comporte, pour chacun des points de trajectoire mémorisés dans la mémoire TRAJ 5 (ou pour chacun d'un sous-ensemble de points de trajectoire mémorisés dans la mémoire TRAJ 5), la définition d'une ou de quelques sous-sections d'une image de la trajectoire capturée en ledit point considéré (la réunion des sous-sections étant strictement inférieure à la totalité de l'image).

Pour chacune des sous-sections associées à un point de trajectoire est en outre associée au moins une information respective, relative à la capture d'images en ce point de trajectoire ou au traitement des images capturées en ce point ainsi qu'optionnellement des données extérieures (données météorologiques, données trafic ferroviaire, signalement de danger sur la voie, etc.)

Par exemple, une sous-section délimite la portion de trajectoire à suivre par le train (i.e. dans le cas du train, la portion de voie ferrée) et figurant dans l'image, ou au moins la portion la plus proche du train de cette trajectoire, et l'isole ainsi du reste de l'image : une telle sous-section correspond à la sous-section 30 sur l'image représentée en figure 3.Par exemple, une sous-section délimite l'entrée ou la sortie d'un tunnel par lequel la trajectoire passe : une telle sous-section correspond à la sous-section 31 sur l'image représentée en figure 3.

Par exemple, une sous-section délimite une zone au sein de laquelle un danger peut survenir (présence d'objets ou d'individus pouvant nécessiter un ralentissement du train, voire un freinage d'urgence), par exemple délimitant les abords immédiats de la trajectoire au niveau du point considéré : une telle sous-section correspond par exemple à la sous-section 32, ou 34 sur l'image représentée en figure 3.

Par exemple, la sous-section 35 de la figure 3 correspondant à la partie supérieure de l'extrémité du tunnel sous laquelle le train va prochainement passer est une zone de danger connue pour ses risques d'éboulements ; c'est également une zone de l'image, suivant l'heure du passage du train, qui est propre à être exposée à un soleil couchant, objet d'éblouissement engendrant la saturation du capteur caméra 711. Le bloc de contrôle 2 est adapté pour déterminer des valeurs de paramètres, comme cela sera décrit plus loin et pour commander la mise en oeuvre desdites valeurs déterminées au module de délivrance de données notamment d'images 7.

Le module de délivrance de données 7 comprend un ensemble 71 de capteur(s) et un bloc de traitement de données capturées 72.

L'ensemble de capteur(s) 71 comprend trois capteurs de données d'images (une caméra 711, un Lidar 712, un Radar 713). Le module de délivrance de données 7 est un module de délivrance de données capturées par les capteurs d'images et bloc de traitement de données 72 est un bloc de traitement des données c'est-à-dire des images capturées.

Les capteurs d'images (par exemple dans l'exemple considéré, la caméra 711, est de type LCD ou autre, le capteur d'image Lidar 712 est un capteur basé sur l'écho de faisceaux lasers, le capteur Radar 713 est un capteur basé sur l'écho d'ondes radios) sont adaptés pour capturer, à chaque instant d'acquisition Tn (par exemple toutes les MS millisecondes, avec MS compris dans une plage de valeurs de [100 à 1500], ajustable en fonction de la vitesse de déplacement du train et/ou des paramètres extérieurs environnementaux, ou en une pluralité de positions géographiques prédéterminées, (par exemple correspondant aux positions géographiques mémorisées dans la base de données CARTO 61), une image de la zone géographique correspondant à la zone d'acquisition du capteur et pour délivrer l'image capturée au bloc de traitement d'images 72.

La caméra 711 comprend des moyens de réglage de valeurs de paramètres de capture d'image. Ces paramètres de capture d'images comprennent par exemple : la distance de focalisation de la caméra et/ou l'aire de focalisation et/ou le contraste et/ou la balance des blancs et/ou des filtres de traitement.

Le LIDAR 712 et RADAR 713 comprennent des moyens de réglage de valeurs de paramètres de capture d'image. Ces paramètres de capture d'images comprennent par exemple : le type de balayage, les zones ciblées, les fréquences utilisées et la portée.

La caméra 711, le LIDAR 712 et le RADAR 713 sont, dans l'exemple considéré, fixés à l'avant du train et capturent donc des images d'une zone de l'environnement faisant face au train dans son sens de marche. Certains autres éléments du système 1 d'acquisition d'images sont, suivant les modes de réalisation, également embarqués ou non. Les échanges de données entre les différents éléments du système 1 d'acquisition ont lieu par l'intermédiaire de liens de télécommunication entre ces éléments, filaires ou sans fil, notamment radiofréquences.

Le bloc de traitement 72 est adapté pour effectuer des traitements sur les images délivrées par les capteurs 711, 712, 713, en fonction de valeurs de paramètres définissant le(s) traitement(s) effectué(s). Ces traitements comportent, sélectivement sur des sous-sections de l'image : la détection de la présence d'objets, l'identification du type d'objets détectés, l'évaluation de la distance à un objet détecté etc. Les paramètres définissent par exemple quelle est la sous-section concernée par le traitement à appliquer, ou quelles sont les sous-sections concernées, quel(s) traitement(s) respectif(s) sont à réaliser sur chacune de ces sous-sections (des traitements distincts étant en effet appliqués sur des sous-portions distinctes d'une image).

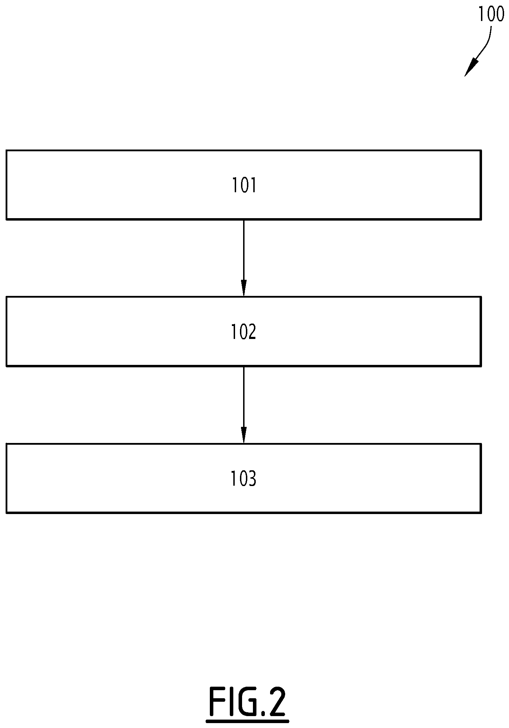

Dans le mode de réalisation considéré, le bloc de contrôle 2 comprend une mémoire 3 et un microprocesseur 4 ; la mémoire 3 stocke des instructions logicielles qui, lors de leur exécution par le microprocesseur 4, mettent en oeuvre les étapes décrites ci-dessous à la figure 2 et incombant au bloc de contrôle 2.

Dans un autre mode de réalisation, le bloc de contrôle 2 est réalisé sous forme d'un composant logique programmable, tel qu'un FPGA (de l'anglais Field Programmable Gate Array), ou encore sous forme d'un circuit intégré dédié, tel qu'un ASIC (de l'anglais Applications Spécifie Integrated Circuit).

La figure 2 présente un organigramme d'étapes mises en oeuvre dans un mode de réalisation de l'invention.

Dans une étape 101, le bloc de contrôle 2 détermine la position courante du train (par exemple celle du point en lequel se trouve à ce moment même la caméra 711, le Lidar 712 et le Radar 713 installés sur le train), appelé point P1 ci-dessous.

Cette position peut être déterminée de différentes façons : par un capteur GPS disposé sur le train et/ou par traitement d'images par le bloc de traitement 72 et/ou par le bloc de contrôle 2 en fonction de la trajectoire prédéterminée indiquée dans la base CARTO 61 et des vitesses instantanées du train, etc.

En fonction de la position courante P1 ainsi déterminée, de la mémoire TRAJ 5 et de la base de données CARTO 61, le bloc de contrôle 2 détermine quel est prochain point de trajectoire P2 présent dans la base CARTO 61 en lequel le train va passer.

Dans une étape 102, le bloc de contrôle 2 extrait de la base de données CARTO 61 la définition des sous-section d'intérêt associées au prochain point de trajectoire P2, ainsi que les informations associées à ces sous-sections d'intérêt figurant dans la base CARTO 61.

Pour chacune de ces sous-sections, le bloc de contrôle 2 détermine à partir de l'information associée à la sous-section, une valeur d'au moins un paramètre de capture d'image pour au moins un des capteurs 711, 712, 713 audit prochain point de trajectoire P2 et une valeur de paramètre de traitement par le bloc de traitement 72 de l'image capturée en ledit point de trajectoire P2 par au moins l'un de ces capteurs.

Dans une étape 103, le bloc de contrôle 2 transmet un message de commande au module de délivrance d'images 7 indiquant au moins ladite valeur de paramètre de capture d'image pour au moins un des capteurs 711,712,713 audit prochain point de trajectoire P2 et ladite valeur de paramètre de traitement. Le module de délivrance d'images 7 les fournit ensuite à celui ou ceux des capteurs 711,712,713 concerné(s) (s'il s'agit d'une valeur de paramètre de capture d'image) ou au bloc de traitement 72 (s'il s'agit d'une valeur de paramètre de traitement d'image).

Lors du passage audit prochain point de trajectoire P2, les capteurs d'image 711, 712, 713 capturent alors une image telle que la valeur de chaque paramètre ayant fait l'objet d'une commande pour ce point de trajectoire P2 par le bloc de contrôle 2 est fixée à la valeur commandée.

Chaque image capturée audit point de trajectoire P2 est ensuite fournie au bloc de traitement 72, qui la traite en fonction de valeurs de paramètres de traitement telle que la valeur de chaque paramètre ayant fait l'objet d'une commande pour ce point de trajectoire P2 par le bloc de contrôle 2 est fixée à la valeur commandée par ce dernier relativement au point P2.

Ces opérations sont réalisées en temps réel.

Les applications sont variées.

Les images ainsi traitées sont ensuite utilisées pour déclencher certaines actions : aide au système de régulation de vitesse (accélération, décélération, ...), arrêt d'urgence, transmission d'alertes à des destinataires distants pour intervention (centre opérationnel, pompier, police, etc.),

Selon les modes de réalisation, seules les sous-sections indiquées dans la base CARTO 61, ou encore seules celles des sous-sections particulières d'image correspondant à un certain type d'informations, bénéficient d'un traitement (par exemple de détection d'obstacles) par le bloc de traitement 72, par exemple les zones 30, 31, 32, 34, 35, le reste de l'image n'étant pas traité (ou avec une priorité nettement plus faible).

Par exemple, dans la plupart des points de la trajectoire, seule la sous-section de trajectoire bénéficiera d'un traitement combiné de la caméra pour la détection d'obstacle frontale immédiate sur la voie, du radar pour la détection d'obstacle longue portée et du lidar pour la détection obstacle latéral moyenne portée ; tandis que, dès qu'il y a approche d'une entrée d'un tunnel, outre la sous-section de trajectoire, les sous-sections correspondant aux côtés de la voie seront aussi prises en charge dans le traitement de l'image de la caméra .La partie supérieure du tunnel, dont l'image fortement saturée par le soleil sera exclue du traitement de l'image de la caméra et la sous-section sera traitée principalement par un balayage adapté du Lidar ou Radar en fonction de la distance au danger. Cette disposition permet de réduire les temps de traitement et/ou les ressources de calcul nécessaires au traitement, d'utiliser au mieux les systèmes de captures de données/d'images embarqués en les adaptant notamment aux données contextuelles et de traiter en priorité que les sous-sections utiles.

Les traitements sont différents sur des sous-sections différentes, et le bloc de contrôle 2 détermine le traitement à effectuer sur chaque sous-portion en fonction de l'information qui est associée à la sous-section dans la base CARTO 61 : par exemple lors de l'arrivée en gare, à proximité d'un quai, les sous-sections (ou zones dites) d'intérêt seront le quai droit ou gauche (fonction de la position de la voie ferrée) où sont positionnés les passagers et les zones de chute d'objet ou de personne sur la voie. Le lidar sera principalement utilisé pour identifier un mouvement de chute (sur la sous-portion correspondant au bord de quai), la caméra sera mise à contribution pour identifier et reconnaître un objet sur la voie (sur la sous-section de voie) et dans le gabarit du passage du train (cette stratégie pourra évoluer en fonction des informations extérieures météos (exposition par un soleil de face), de la possibilité de récupérer des images de caméras fixes à quai, (ou de trains voisins ou arrivant à proximité) pour traiter des zones d'intérêts complexes et difficiles à analyser depuis le train.

Dans un autre exemple, lors de l'approche du train d'un passage à niveau, les sous-sections d'intérêt seront les portions latérales de la route traversant la voie ferrée. La priorité maximale sera mise en place pour détecter un objet présent ou croisant la route et qui pourrait entrer en collision avec le train. La distance entre la route et le train étant parfaitement connue (CARTO 61), la configuration de portée ainsi que l'angle de balayage radar et lidar seront configurés pour couvrir au mieux les sous-sections concernées. La sous-section d'intérêt de la caméra 711 se fera principalement sur la détection d'objet ou personne immédiate sur la voie. L'unité de traitement 72, vérifiera la possibilité d'alerte au centre opérationnel d'exploitation afin de déterminer si une zone de danger supplémentaire doit être considérée dans l'analyse. Cette stratégie peut être adaptée en fonction des informations extérieures météos (exposition soleil de face), de la possibilité de récupérer des caméras fixes sur le passage à niveau, (ou de trains voisins ou arrivant à proximité) pour traiter des zones d'intérêts complexes et difficiles à analyser depuis le train.

Dans des modes de réalisation, le bloc de contrôle 2 déterminera en fonction de l'information associée dans la base CARTO 61 et/ou de données extérieures (données météorologiques, données trafic ferroviaire, signalement de danger sur la voie, etc.) les valeurs de contraste (et/ou de saturation), de balayage radar, lidar (et/ou portées) fixées pour les différentes sous-section d'intérêts à traiter au point P2 correspondant par exemple à l'image de la figure 3 pour chaque système d'acquisition, c'est-à-dire la caméra 711, Lidar 712 et radar 713. Une telle disposition aura pour effet que la zone de trajectoire dans le tunnel sera exploitable pour traitement par le bloc de traitement 72, alors que si la valeur de contraste avait été décidée à partir de l'ensemble de la zone d'acquisition, la portion de trajectoire située dans le tunnel serait apparue noire dans le noir correspondant au tunnel et aurait été inexploitable par traitement d'image). Cette disposition permet également de régler au plus juste l'angle de balayage radar et lidar pour sonder uniquement l'intérieur du tunnel (longue portée) et la partie supérieure du tunnel et latérales (courtes portées) améliorant ainsi la résolution des images dans les temps de mesures impartis à l'acquisition. Cette disposition permet notamment de rendre parfaitement exploitables les images tant à l'abord de l'entrée d'un tunnel qu'à l'abord de la sortie d'un tunnel.

Dans un mode de réalisation, d'autres données complémentaires sont utilisées en fonction de la position : ainsi des sources de données extérieures alimentent des bases de données 61 autres que la base CARTO 61 de l'ensemble de bases de données 6, qui comporte alors par exemple une base de données météorologiques, une base de données relative à la pollution, une base de données relative au trafic etc. Des données complémentaires relatives au train telles que vitesse du train, charge du train peuvent en outre être utilisées. Et dans un mode de réalisation de l'invention, à l'étape 102, le bloc de contrôle 2 extrait de la base de données CARTO 61 la définition de données complémentaires de météo, pollution, trafic, etc. associées au prochain point de trajectoire P2 et de vitesse, et le bloc de contrôle 2 détermine en outre la valeur de paramètres de capture des images et/ou la valeur de paramètres de traitement d'images en fonction de ces données complémentaires relatives au point P2. Par exemple, alors que le train roule encore dans un tunnel et approche de la sortie du tunnel, le bloc de contrôle 2 peut ainsi déterminer la météo qu'il fera à la sortie du tunnel et décider du filtre à appliquer par la caméra en fonction de la météo.

Dans un mode de réalisation, une sous-section de l'image peut être définie comme sous-section à exclure du traitement par le bloc de traitement 72 ou à exclure de la zone de définition de la valeur d'un paramètre de capture (correspondant par exemple à la localisation d'objets brillants, de surfaces réflectives ...), de manière dynamique ou fixe. Par exemple, à l'étape 102, le bloc de contrôle 2 détermine en fonction de données complémentaires qu'à la sortie du tunnel, lors de la capture d'image au point P2, le soleil sera face à la caméra et présent dans l'image. Il est alors adapté pour délimiter une sous-portion de l'image prise au point P2 comprenant le soleil, pour commander à la caméra d'exclure cette sous-portion de la zone d'acquisition à partir de laquelle la caméra 711 détermine la valeur des paramètres de capture et/ou pour commander au bloc de traitement 72 d'exclure cette sous-portion du traitement. Une analyse complémentaire par radar ou lidar ou autre système d'acquisition alternatif (qui ne serait pas altéré par la luminosité de l'objet perturbateur) peut être commandée par le bloc de traitement 72 si la zone exclue fait partie dans une sous-portion dite d'intérêt. Cette analyse complémentaire peut-être aussi commandée à un système d'acquisition externe, s'il est accessible par télécommunication, par exemple une caméra de voie située à proximité couvrant la dite sous-portion d'intérêt.

Dans un mode de réalisation, pour un passage à niveau, il y a du brouillard, le bloc de contrôle 2 détermine que la caméra frontale (ou le LIDAR ou RADAR) ne peut être utilisée pour vérifier qu'il n'y a pas de voiture sur les voies à 150m ; il se connecte alors, par télécommunication, à la caméra fixe du passage à niveau pour récupérer l'image et réaliser le traitement de détection d'obstacle.

Ou encore, dans un mode de réalisation, le bloc de contrôle 2 requiert la réception, par voie de télécommunication sans fil, des données captées par une caméra de voie en sortie de tunnel, pour traiter les dangers en sortie de tunnel qui sont inaccessibles tant que le train est dans le tunnel.

La présente invention permet ainsi suivant les modes de réalisation de focaliser dynamiquement l'effort d'analyse d'images sur des portions réduites de chaque acquisition (celles d'intérêt) afin de diminuer le temps et/ou les ressources de traitement. Par exemple, dans le cas d'un métro, entre deux stations de métro, seule la sous-portion de trajectoire sera traitée, tandis que lors de l'arrivée en station, les sous-portions correspondant aux quais feront aussi partie des zones traitées.

La présente invention permet suivant les modes de réalisation de focaliser dynamiquement la détermination de valeurs de paramètres de capture, de balayage, de portée des images (caméra, lidar, radar), sur des portions réduites (celles d'intérêt) de la zone d'acquisition des capteurs afin d'optimiser ces valeurs des paramètres de capture au vu de ce qu'il sera utile de traiter.

Ces effets sont particulièrement utiles lorsque les trains, ou plus généralement les engins mobiles mettant en oeuvre l'invention, comportent un module de guidage autonome utilisant les images, capturées par un ensemble de capteur(s) embarqué(s).

Ainsi, dans le mode de réalisation considéré, l'invention propose une interaction en temps réel entre le traitement d'images et l'algorithme de guidage du train pour déterminer une zone particulière de l'image qui doit être optimisée (même si cela dégrade le rendu global du reste de l'image).

Dans des modes de réalisation, la zone d'acquisition du capteur est une zone de l'environnement du train face à la marche du train comme indiqué plus haut ou une autre zone à proximité du train.

L'exemple décrit ci-dessus fait référence au cas d'un train. Bien sûr l'invention est applicable au cas de tout véhicule dont la trajectoire est parfaitement prédéfinie (au moins sur une durée minimum de 5 secondes, cette durée peut être réduite en fonction de la rapidité des unités de calculs et des systèmes de télécommunication à obtenir les données nécessaires à la méthodologie décrite ci-dessus permettant l'identification et le traitement des zones d'intérêts) et dont la position courante sur la trajectoire est connue : métros, tramways, bus et voitures à conduite autonome ou avec module d'assistance à la conduite.

## Revendications

1. Procédé d'acquisition de données, dans un module électronique d'acquisition (1), lesdites données étant capturées par un module de capture de données (71) comprenant trois capteurs de données d'images choisis parmi : une caméra (711), un LIDAR (712) et un RADAR (713) , qui sont embarqués sur un engin mobile et qui délivrent à chaque instant d'acquisition de chaque capteur un ensemble de données capturées correspondant à la zone d'acquisition de chaque capteur ; selon lequel l'engin mobile suit une trajectoire prédéfinie, ledit procédé étant **caractérisé en ce que**, ledit module électronique d'acquisition (1) comprend une base de données (61) définissant, pour chacun d'une pluralité de points de la trajectoire prédéfinie, un ensemble de sous-sections d'intérêt indiquant une pluralité de sous-sections d'intérêt (30, 31) dans la zone d'acquisition de chaque capteur, le procédé comprenant les étapes suivantes mises en oeuvre en temps réel par le module d'acquisition :
- détermination d'une prochaine position de l'engin mobile sur ladite trajectoire prédéfinie de l'engin mobile ;
- extraction de la base de données (61) de la définition de la pluralité de sous-section d'intérêt associées à la prochaine position, ainsi que des informations associées à ces sous-sections d'intérêt figurant dans la base de données (61),
- détermination, pour chacune de ces sous-sections d'intérêt (30, 31), à partir de l'information associée à la sous-section (30, 31), de la valeur d'au moins un paramètre de capture d'image pour chacun des capteurs (711, 712, 713) à ladite prochaine position et d'une valeur de paramètre de traitement de l'image capturée en ladite prochaine position par chacun des capteurs ;
- capture d'une image lors du passage à ladite prochaine position en fonction d'au moins ladite valeur de paramètre de capture d'image pour chacun des capteurs (711, 712, 713) en ladite prochaine position ;
- commande d'une opération de traitement des données capturées par chaque capteur embarqué à ladite prochaine position conformément à la valeur du ou de chaque paramètre de traitement pour chacun des capteurs en ladite prochain position ;
selon lequel pour chaque point de la trajectoire prédéfinie, la réunion des sous-sections d'intérêt est inférieure à la zone d'acquisition de chaque capteur et ledit traitement est réalisé sélectivement sur le sous-ensemble desdites données capturées dans lesdites sous-section d'intérêt,
le traitement réalisé étant différent sur des sous-sections d'intérêt différentes de l'ensemble de sous-sections d'intérêt.

2. Procédé d'acquisition de données selon la revendication 1, selon lequel ladite détermination de la valeur du au moins un paramètre est effectuée en fonction de ladite sous-section d'intérêt (30, 31) et à l'exclusion de sous-sections de la zone d'acquisition non indiquées dans ledit ensemble de sous-section d'intérêt.

3. Procédé d'acquisition de données selon la revendication 1 ou 2, selon lequel une sous-section d'intérêt (30, 31) est une portion de trajectoire à venir.

4. Procédé d'acquisition de données selon l'une des revendications précédentes, selon lequel une sous-section d'intérêt (31) correspond à une extrémité d'un tunnel.

5. Procédé d'acquisition de données selon l'une des revendications précédentes, selon lequel ladite détermination de la valeur du au moins un paramètre est effectuée en outre en fonction de données météorologiques.

6. Procédé d'acquisition de données selon l'une des revendications précédentes, comprenant les étapes suivantes mises en oeuvre par le module d'acquisition (1) :
- identification d'un problème d'exploitation, ou d'incomplétude, de données en provenance de chaque capteur (711), et
- suite à ladite identification, déclenchement de la fourniture de données complémentaires par un système d'acquisition de données non embarqué dans l'engin mobile.

7. Programme d'ordinateur comportant des instructions logicielles qui, lorsqu'elles sont exécutées par un ordinateur, mettent en oeuvre un procédé selon l'une quelconque des revendications précédentes.

8. Module électronique (1) d'acquisition de données capturées par un module de capture de données (71) comprenant trois capteurs de données d'images choisis parmi : une caméra (711), un LIDAR (712) et un RADAR (713), qui délivrent à chaque instant d'acquisition du ou de chaque capteur un ensemble de données capturées correspondant à la zone d'acquisition de chaque capteur et qui sont embarqués sur un engin mobile suivant une trajectoire prédéfinie, ledit module électronique d'acquisition (1) étant **caractérisé en ce qu'**il, comprend une base de données (61) définissant, pour chacun d'une pluralité de points de la trajectoire prédéfinie, un ensemble de sous-sections d'intérêt (30, 31) indiquant une pluralité de sous-sections d'intérêt dans la zone d'acquisition du ou de chaque capteur, la réunion des sous-sections d'intérêt pour chaque point de la trajectoire prédéfinie étant inférieure à la zone d'acquisition du capteur et **en ce qu'**il est adapté pour, en temps réel, déterminer une prochaine position de l'engin mobile sur ladite trajectoire prédéfinie de l'engin mobile, pour extraire de la base de données (61) la définition de la pluralité de sous-sections d'intérêt associées à la prochaine position, ainsi que des informations associées à ces sous-sections d'intérêt figurant dans la base de données (61), et pour déterminer, pour chacune de ces sous-sections d'intérêt, à partir de l'information associée à la sous-section (30, 31), la valeur d'au moins un paramètre de capture d'image pour chacun des capteurs (711, 712, 713) à ladite prochaine position et d'une valeur de paramètre de traitement de l'image capturée en ladite prochaine position par chacun des capteurs , pour capturer une image lors du passage à ladite prochaine position en fonction d'au moins ladite valeur de paramètre de capture d'image pour chacun des capteurs (711,712,713) en ladite prochaine position et pour commander l'opération de traitement conformément à la valeur du ou de chaque paramètre de traitement pour chacun des capteurs en ladite prochain position, et pour effectuer ledit traitement sélectivement sur le sous-ensemble desdites données capturées dans lesdites sous-sections d'intérêt,
le traitement réalisé étant différent sur des sous-sections d'intérêt différentes de l'ensemble de sous-sections d'intérêt.

9. Véhicule adapté pour suivre une trajectoire prédéfinie, comprenant le module électronique (1) d'acquisition de données selon la revendication 8.

## Patentansprüche

1. Datenerfassungsverfahren in einem elektronischen Erfassungsmodul (1), wobei die Daten von einem Datenaufnahmemodul (71) aufgenommen werden, umfassend drei Bilddatensensoren, die ausgewählt sind aus: einer Kamera (711), einem LIDAR (712) und einem RADAR (713), die an Bord einer mobilen Maschine sind und die zu jedem Erfassungszeitpunkt von jedem Sensor einen Satz aufgenommener Daten bereitstellen, der der Erfassungszone von jedem Sensor entspricht; wobei die mobile Maschine einem vordefinierten Streckenverlauf folgt, wobei das Verfahren **dadurch gekennzeichnet ist, dass** das elektronische Erfassungsmodul (1) eine Datenbank (61) umfasst, die für jeden einer Vielzahl von Punkten auf dem vordefinierten Streckenverlauf einen Satz von Teilbereichen von Interesse definiert, der eine Vielzahl Teilbereichen von Interesse (30, 31) in der Erfassungszone von jedem Sensor angibt, das Verfahren umfassend die folgenden Schritte, die von dem Erfassungsmodul in Echtzeit implementiert werden:
- Bestimmen einer nächsten Position der mobilen Maschine auf dem vordefinierten Streckenverlauf der mobilen Maschine;
- Abrufen, aus der Datenbank (61), der Definition der Vielzahl von Teilbereichen von Interesse, die mit der nächsten Position assoziiert sind, sowie der Informationen, die mit diesen Teilbereichen von Interesse assoziiert sind, die in der Datenbank (61) enthalten sind,
- Bestimmen, für jeden dieser Teilbereiche von Interesse (30, 31), aus den Informationen, die mit dem Teilbereich (30, 31) assoziiert sind, des Werts von mindestens einem Bildaufnahmeparameter für jeden der Sensoren (711, 712, 713) an der nächsten Position und eines Verarbeitungsparameterwerts des aufgenommenen Bilds an der nächsten Position für jeden der Sensoren;
- Aufnehmen eines Bilds beim Übergang zu der nächsten Position abhängig von mindestens dem Bildaufnahmeparameterwert für jeden der Sensoren (711, 712, 713) an der nächsten Position;
- Steuern eines Verarbeitungsvorgangs der aufgenommenen Daten für jeden Sensor an Bord an der nächsten Position gemäß dem Wert von dem oder jedem Verarbeitungsparameter für jeden der Sensoren an der nächsten Position;
wobei für jeden Punkt des vordefinierten Streckenverlaufs die Zusammenführung der Teilbereiche von Interesse kleiner ist als die Erfassungszone von jedem Sensor und die Verarbeitung selektiv an dem Teilsatz der Daten durchgeführt wird, die in dem Teilbereich von Interesse aufgenommen werden,
wobei die durchgeführte Verarbeitung auf verschiedenen Teilbereichen von Interesse des Satzes von Teilbereichen von Interesse verschieden ist.

2. Datenerfassungsverfahren nach Anspruch 1, wobei das Bestimmen des Werts des mindestens einen Parameters abhängig von dem Teilbereich von Interesse (30, 31) und unter Ausschluss von Teilbereichen der Erfassungszone, die nicht in dem Satz von Teilbereichen von Interesse angegeben sind, durchgeführt wird.

3. Datenerfassungsverfahren nach Anspruch 1 oder 2, wobei ein Teilbereich von Interesse (30, 31) ein Abschnitt eines bevorstehenden Streckenverlaufs ist.

4. Datenerfassungsverfahren nach einem der vorherigen Ansprüche, wobei ein Teilbereich von Interesse (31) einem Ende eines Tunnels entspricht.

5. Datenerfassungsverfahren nach einem der vorherigen Ansprüche, wobei das Bestimmen des Werts des mindestens einen Parameters ferner abhängig von meteorologischen Daten durchgeführt wird.

6. Datenerfassungsverfahren nach einem der vorherigen Ansprüche, umfassend die folgenden Schritte, die von dem Erfassungsmodul (1) implementiert werden:
- Identifizieren eines Problems bei der Auswertung oder der Unvollständigkeit der Daten von jedem Sensor (711), und
- im Anschluss an die Identifizierung Auslösung, Auslösen der Bereitstellung zusätzlicher Daten durch ein Datenerfassungssystem, das nicht an Bord der mobilen Maschine ist.

7. Computerprogramm, umfassend Softwareanweisungen, die, wenn sie von einem Computer ausgeführt werden, ein Verfahren nach einem der vorherigen Ansprüche implementieren.

8. Elektronisches Modul (1) zum Erfassen von Daten, die von einem Datenaufnahmemodul (71) aufgenommen werden, umfassend drei Bilddatensensoren, die ausgewählt sind aus: einer Kamera (711), einem LIDAR (712) und einem RADAR (713), die zu jedem Erfassungszeitpunkt von dem oder jedem Sensor einen Satz aufgenommener Daten bereitstellen, die der Erfassungszone von jedem Sensor entsprechen, und die an Bord einer Maschine sind, die einem vordefinierten Streckenverlauf folgt, wobei das elektronische Erfassungsmodul (1) **dadurch gekennzeichnet ist, dass** es eine Datenbank (61) umfasst, die für jeden einer Vielzahl von Punkten auf dem vordefinierten Streckenverlauf einen Satz von Teilbereichen von Interesse (30, 31) definiert, die eine Vielzahl von Teilbereichen von Interesse in der Erfassungszone des oder jedes Sensors angeben, wobei die Zusammenführung der Teilbereiche von Interesse für jeden Punkt des vordefinierten Streckenverlaufs kleiner ist als die Erfassungszone des Sensors, und dass es für Folgendes in Echtzeit angepasst ist Bestimmen einer nächsten Position der mobilen Maschine auf dem vordefinierten Streckenverlauf der mobilen Maschine, um aus der Datenbank (61) die Definition der Vielzahl von Teilbereichen von Interesse, die mit der nächsten Position assoziiert sind, sowie Informationen, die mit diesen Teilbereichen von Interesse assoziiert sind, die in der Datenbank (61) enthalten sind, zu extrahieren, und Bestimmen, für jeden dieser Teilbereiche von Interesse, anhand der mit dem Teilbereich (30, 31) assoziierten Informationen den Wert mindestens eines Bildaufnahmeparameters für jeden der Sensoren (711, 712, 713) an der nächsten Position und einen Verarbeitungsparameterwert an der nächsten Position von jedem der Sensoren aufgenommenen Bilds zu bestimmen, Aufnehmen eines Bilds beim Übergang zu der nächsten Position abhängig von mindestens dem Bildaufnahmeparameterwert für jeden der Sensoren (711, 712, 713) an der nächsten Position und Steuern des Verarbeitungsvorgangs gemäß dem Wert des oder jedes Verarbeitungsparameters für jeden der Sensoren an der nächsten Position, und Durchführen der Verarbeitung selektiv an dem Teilsatz der aufgenommenen Daten in den Teilbereichen von Interesse,
wobei die durchgeführte Verarbeitung auf verschiedenen Teilbereichen von Interesse des Satzes von Teilbereichen von Interesse verschieden ist.

9. Fahrzeug, das angepasst ist, um einem vordefinierten Streckenverlauf zu folgen, umfassend das elektronische Modul (1) zur Datenerfassung nach Anspruch 8.

## Claims

1. A data acquisition method, in an electronic acquisition module (1), said data being detected by a data capture module (71) comprising three image data sensors chosen from: a camera (711), a LIDAR (712) and a RADAR (713), which are embedded on a moving vehicle and which deliver, at each acquisition moment of each sensor a set of captured data corresponding to the acquisition zone of each sensor; according to which the moving vehicle follows a predefined trajectory, said method being **characterized in that** said electronic acquisition module (1) comprises a database (61) defining, for each of a plurality of points of the predefined trajectory, a set of subsections of interest indicating a plurality of subsections of interest (30, 31) in the acquisition zone of each sensor, the method comprising the following steps implemented in real time by the acquisition module:
- determining a next position of the moving vehicle on said predefined trajectory of the moving vehicle;
- extraction from the database (61) of the definition of the subsections of interest associated with the next position, as well as the information associated with these subsections of interest appearing in the database (61),
- determining, for each of these subsections of interest (30, 31), from the information associated with the subsection (30, 31), the value of at least one image capture parameter for each of the sensors (711, 712, 713) at said next position and a parameter value for processing the image captured at said next position by each of the sensors;
- capturing an image during the passage to said next position as a function of at least said image capture parameter value for each of the sensors (711, 712, 713) at said next position;
- controlling an operation of processing parameters of the data captured by each embedded sensor in the next position according to the value of the or each processing parameter for each of the sensors in said next position;
according to which for each point of the predefined trajectory, the union of the subsections of interest is less than the acquisition zone of each sensor and the said processing is done selectively over the subset of said captured data in said subsections of interest,
the processing operation being different on different subsections of the set of subsections of interest.

2. The data acquisition method according to claim 1, wherein said determination of the value of the parameters is done as a function of said at least first and second subsections of interest (30, 31) and to the exclusion of subsections of the acquisition zone not indicated in said subsection of interest subset.

3. The data acquisition method according to one to claim 1 or 2, wherein a subsection of interest (30, 31) is an upcoming trajectory portion.

4. The data acquisition method according to one of the preceding claims, wherein a subsection of interest (31) corresponds to one end of a tunnel.

5. The data acquisition method according to one of the preceding claims, according to which said determination of the value of the at least one parameter is further done as a function of meteorological data.

6. The data acquisition method according to one of the preceding claims, comprising the following steps carried out by the acquisition module (1):
- identification of an exploitation problem, or incompleteness, of data coming from each sensor (711), and
- following said identification, triggering of the supply of complementary data by a data acquisition system not embedded in the moving vehicle.

7. A computer program comprising software instructions which, when executed by a computer, carry out a method according to any one of the preceding claims.

8. An electronic module (1) for acquiring data detected by a data capture module (71) comprising three image data sensors chosen from: a camera (711), a LIDAR (712) and a RADAR (713) which deliver, at each acquisition instant of each sensor, a set of captured data corresponding to the acquisition zone of each sensor and which are embedded on a moving vehicle following a predefined trajectory, said electronic acquisition module (1) being **characterized in that** it comprises a database (61) defining, for each of a plurality of points of the predefined trajectory, a set of subsections of interest indicating a plurality of subsections of interest (30, 31) in the acquisition zone of each sensor, the union of the subsections of interest for each point of the predefined trajectory being smaller than an acquisition zone of the sensor and **in that** it is suitable for determining, in real time, a next position of the moving vehicle on said predefined trajectory of the moving vehicle, and for determining, as a function of at least the subsection of interest defined by the database for the point corresponding to the determined position, the value of at least one image capture parameter for each of the sensors (711, 712, 713) at said next position and a parameter value for processing the image captured at said next position by each of the sensors, for capturing an image during the passage to said next position as a function of at least said image capture parameter value for each of the sensors (711, 712, 713) at said next position and for controlling the operation among the detection and the processing according to the value of the or each processing parameter for each of the sensors in said next position, and for performing said processing of the detected data.

9. A vehicle suitable for following a predefined trajectory, comprising the electronic data acquisition module (1) according to claim 8.
